# EUROPEAN PATENT SPECIFICATION

(11) **EP 3 965 834 B1**
(45) Date of publication and mention of the grant of the patent: **24.01.2024**
(21) Application number: 19719760.1
(22) Date of filing: 06.05.2019
(51) Int. Cl.: A61L 9/01, A61K 8/49, A61Q 13/00, A61Q 15/00

(54) **USE OF A MALODOR COUNTERACTING COMPOSITION AND METHOD THEREOF**
VERWENDUNG EINER GERUCHSKONTROLLIERENDEN ZUSAMMENSETZUNG UND VERFAHREN
UTILISATION D'UNE COMPOSITION CONTRE LES MAUVAISES ODEURS ET MÉTHODE ASSOCIÉE

(43) Date of publication of application: 16.03.2022
(73) Proprietor: ChemCom S.A., 1070 Brussels (BE)
(72) Inventor: VEITHEN, Alex, 1476 Genappe (BE); CHATELAIN, Pierre, 1150 Brussels (BE); QUESNEL, Yannick, 1300 Wavre (BE)
(74) Representative: De Clercq & Partners
(86) International application number: PCT/EP2019/061513
(87) International publication number: WO 2020/224754

(56) References cited:
- WO-A1-2016/154503
- US-A1- 2006 160 789

## Description

### Filed of the invention

The present invention relates to a malodor or off-taste counteracting composition. In particular the invention relates to an attenuator or suppressor earthy, musty and/or moldy malodors or off-tastes. The invention also relates to the use of said composition and attenuator or suppressor, for attenuating or suppressing malodors or off-tastes, in particular earthy, musty and/or moldy malodors and off-tastes.

### Background of the invention

The olfactory earthy note is described as the smell of a freshly plunged soil. Although it may be a component of some smells usually considered as pleasant, such as petrichor (the typical smell that accompanies the first rain after a spell of warm weather), it is also constitutive of mildew or moldy or musty odors that are rather quoted as unpleasant.

The earthy note can be elicited by different chemicals, among which geosmin ((4S,4aS,8aR)-4,8a-Dimethyl-1,2,3,4,5,6,7,-octahydronaphthalen-4a-ol, C₁₂H₂₂O, CAS 19700-21-1) is probably the most typical. Other naturally occurring molecules, such as, but not limited to, 2-methylisoborneol, 2-ethylfenchol, huminol, ambrinol or synthetic compounds such, as but not limited to, terranol also possess the typical earthy note. Likewise, more complex mixtures, such patchouli oil disclose an earthy aspect. These different compounds and mixtures may possess additional olfactory notes described either as camphoraceous, terpenic, woody, amberry, musky, with respect to the considered compound or mixture.

They earthy note elicited by natural or synthetic products may correspond to a problem in different contexts. Two compounds, geosmin and 2-methylisobomeol, are often reported as the main culprits of mildew malodor or off-taste. The human nose is particularly sensitive to these compounds, with a detection threshold as low as 10 ng/L and 25 ng/L for geosmin and 2-methylisoborneol respectively. They occur as metabolites of Streptomycetes, cyanobacteria or molds that grow in damp environments or directly in water. Both compounds are not considered as toxic by themselves, but their presence is interpreted as a clue of contamination. They are responsible of the typical musty/moldy odor found in dank cellars or in buildings that present a problem of humidity and are therefore considered as an olfactory nuisance.

Both molecules may also be responsible of an off-taste in drinking water contained in reservoir or ducts. Even after a water cleaning step that would have removed biological contaminants, the remaining geosmin and/or 2-methylisoborneol could still have a repulsive effect on human consumers. Contaminations with these earthy smelling molecules may also occur during the fabrication process of other beverages such as wine or beer, conferring to the product an unpleasant off-taste. Likewise, geosmin and 2-methylisoborneol may accumulate in the flesh of fishes raised in pond, giving a taint that makes the fish meat unacceptable for consumers, although it represents a valuable food source.

Other compounds that are used in perfumery also possess the typical earthy odorant note along with other hues that are more interesting for perfume compositions. The earthy note can therefore constitute a limitation for their use in fragrance creation.

US 2006/160789 A1 discloses the use of at least one compound of a general formula (I): 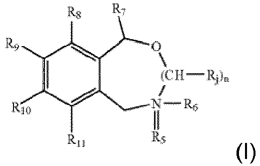 or its pharmaceutically acceptable salts, for the preparation of a medicament intended for the prevention or treatment of acute tolerance to morphines or hyperalgesia caused by morphines, in an individual having received an administration of a morphine analgesic.

WO 2016/154503 A1 discloses oral pharmaceutical compositions suitable for chewing, sucking, or buccal dissolution comprising soft gel capsules and liquid fills, methods for making the same, and methods for treating subjects in need thereof with such capsules. In particular, oral pharmaceutical compositions comprising chewable, suckable, or dissolvable soft gel capsules with various flowable fill compositions are described.

It is therefore of interest to identify means to counteract the unpleasant odorant character elicited by earthy olfactory note.

### Summary of the invention

The present inventors have surprisingly found that a compound previously described as a potent analgesic (Cohen and Hernandez, J. Int Med Res, 1976, 4, pp 138-143) namely nefopam can be used as a malodor counteracting agent, in particular for counteracting earthy, musty and/or moldy malodors, or earthy, musty and/or moldy off-tastes.

In a first aspect, the present invention relates to the use, as defined in the appended set of claims, of racemic nefopam free base, ((-)-nefopam free base, or a mixture thereof, or a polymorph or a solvate thereof, or use of a composition comprising racemic nefopam free base, ((-)-nefopam free base, or a mixture thereof, or a polymorph or a solvate thereof, as a malodor counteracting agent, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

In a second aspect, the present invention provides a method of counteracting malodors, as defined in the appended set of claims, comprising: contacting a situs comprising a malodor or a situs that will become malodorous with racemic nefopam free base, ((-)-nefopam free base, or a mixture thereof, or a polymorph or a solvate thereof, or contacting a situs comprising a malodor or a situs that will become malodorous with a composition comprising racemic nefopam free base, ((-)-nefopam free base, or a mixture thereof, or a polymorph or a solvate thereof, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

In a third aspect, the present invention relates to the use of a consumer, industrial or textile product, as defined in the appended set of claims, to freshen, reduce, suppress or eliminate malodors, wherein said product comprises racemic nefopam free base, ((-)-nefopam free base, or a mixture thereof, or a polymorph or a solvate thereof, or a composition comprising racemic nefopam free base, ((-)-nefopam free base, or a mixture thereof, or a polymorph or a solvate thereof, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

The independent and dependent claims set out particular and preferred features of the invention. Features from the dependent claims may be combined with features of the independent or other dependent claims as appropriate.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### Detailed description of the invention

When describing the compounds and processes of the invention, the terms used are to be construed in accordance with the following definitions, unless a context dictates otherwise.

As used in the specification and the appended claims, the singular forms "a", "an," and "the" include both singular and plural referents unless the context clearly dictates otherwise. By way of example, "a compound" means one compound or more than one compound.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The term "about" as used herein when referring to a measurable value such as a parameter, an amount, a temporal duration, and the like, is meant to encompass variations of +/-10% or less, preferably +/-5% or less, more preferably +/-1 % or less, and still more preferably +/-0.1 % or less of and from the specified value, insofar such variations are appropriate to perform in the disclosed invention. It is to be understood that the value to which the modifier "about" refers is itself also specifically, and preferably, disclosed.

As used herein, the term "and/or," when used in a list of two or more items, means that any one of the listed items can be employed by itself or any combination of two or more of the listed items can be employed. For example, if a list is described as comprising group A, B, and/or C, the list can comprise A alone; B alone; C alone; A and B in combination; A and C in combination, B and C in combination; or A, B, and C in combination.

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

Preferred statements (features) and embodiments of the compositions, compounds, uses, and methods of this invention are set herein below. Each statements and embodiments of the invention so defined may be combined with any other statement and/or embodiments unless clearly indicated to the contrary. In particular, any feature indicated as being preferred or advantageous may be combined with any other feature or features or statements indicated as being preferred or advantageous.

The invention comprises the use of racemic nefopam free base, ((-)-nefopam free base, or a mixture thereof, or a polymorph or a solvate thereof, or use of a composition comprising racemic nefopam free base, ((-)-nefopam free base, or a mixture thereof, or a polymorph or a solvate thereof, as a malodor counteracting agent, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

The present invention concerns the use of racemic nefopam free base, ((-)-nefopam free base, or a mixture thereof, or a polymorph or a solvate thereof, to modify, suppress, reduce, decrease, mask and/or block the sensory perception of malodors wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

In an embodiment of the use as described hereinabove, said malodor is earthy, musty and/or moldy malodor.

In an embodiment of the use as described hereinabove, said malodor is earthy malodor.

In an embodiment of the use as described hereinabove, nefopam is (*R* )-5-methyl-1-phenyl-1,3,4,6-tetrahydro-2,5-benzoxazocine free base.

In an embodiment of the use as described hereinabove, the malodor causing agent comprises geosmin.

In an embodiment of the use as described hereinabove, the malodor causing agent comprises 2-methylisoborneol.

The present invention also concerns a method of counteracting malodors comprising: contacting a situs comprising a malodor or a situs that will become malodorous with a counteracting effective amount of racemic nefopam free base, ((-)-nefopam free base, or a mixture thereof, or a polymorph or a solvate thereof, or contacting a situs comprising a malodor or a situs that will become malodorous with a composition comprising racemic nefopam free base, ((-)-nefopam free base, or a mixture thereof, or a polymorph or a solvate thereof, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

The present invention also concerns a method to modify, suppress, reduce, decrease, mask and/or block the sensory perception of malodors, which comprise the step of contacting a situs with racemic nefopam free base, ((-)-nefopam free base, or a mixture thereof, or a polymorph, or a solvate thereof, or with a composition comprising racemic nefopam free base, ((-)-nefopam free base, or a mixture thereof, or a polymorph or a solvate thereof, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

In an embodiment of the method as described hereinabove, said situs is selected from air, a surface, a liquid, or a source of malodors, preferably said situs is air.

In an embodiment of the method as described hereinabove, said malodor is earthy, musty and/or moldy malodor.

The invention also concerns a method for counteracting earthy, musty and/or moldy malodors comprising the use of racemic nefopam free base, ((-)-nefopam free base, or a mixture thereof, or a polymorph or a solvate thereof, or of a composition comprising racemic nefopam free base, ((-)-nefopam free base, or a mixture thereof, or a polymorph or a solvate thereof, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

In an embodiment of the method as described hereinabove, said malodor is earthy malodor.

In an embodiment of the method as described hereinabove, (RS)-5-methyl-1-phenyl-1,3,4,6-tetrahydro-2,5-benzoxazocine free base (CAS registry number 13669-70-0) is used.

In an embodiment of the method as described hereinabove, (R)-5-methyl-1-phenyl-1,3,4,6-tetrahydro-2,5-benzoxazocine free base (CAS registry number 91463-82-0) is used.

In an embodiment of the method as described hereinabove, the malodor causing agent comprises geosmin.

In an embodiment of the method as described hereinabove, the malodor causing agent comprises 2-methylisoborneol.

The invention also concerns the use of a consumer, industrial or textile product to freshen, reduce, suppress or eliminate malodors, wherein said product comprises racemic nefopam free base, ((-)-nefopam free base, or a mixture thereof, or a polymorph or a solvate thereof, or a composition comprising racemic nefopam free base, ((-)-nefopam free base, or a mixture thereof, or a polymorph or a solvate thereof, as malodor counteracting agent, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

In an embodiment of the use as described hereinabove, the consumer, industrial or textile product is used as an air-freshener.

In a first aspect, the invention provides the use of racemic nefopam free base, ((-)-nefopam free base, or a mixture thereof, or the use of a malodor and/or off-taste counteracting composition comprising racemic nefopam free base, ((-)-nefopam free base, or a mixture thereof, or a polymorph or a solvate thereof, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

Nefopam is a known non-opioid, non-steroidal, centrally acting analgesic drug that is derivative of the non-sedative benzoxazocine, developed and known in 1960s as Fenazocine (Bassett JR, Cairncross KD, Hacket NB, Story M.; Br J Pharmacol. 1969; 37:69-78). In the early 1970s, nefopam was developed as an antidepressant and was also used as a myorelaxant for the treatment of spasticity. The additional analgesic property was soon recognized, and although the mechanism of analgesia is not completely understood, it appears that nefopam is a centrally acting, non-opioid analgesic that inhibits reuptake of serotonin, norepinephrine, and dopamine. As a consequence, nefopam has been used extensively in many countries for the treatment of acute and chronic malignant and non-malignant pain. In addition, nefopam is generally considered to be safe and well tolerated.

The present composition comprising racemic nefopam free base, ((-)-nefopam free base, or a mixture thereof, as described herein above, or a polymorph or a crystal habit thereof, or a solvate thereof, is a malodor counteracting composition and is therefore non-therapeutic.

The term "free base" herein to refer to nefopam as distinct from any salt thereof, i.e. devoid, or essentially devoid, of addition salts of nefopam.

Racemic nefopam free base can be used (compound of formula (I): ((RS)-5-methyl-1-phenyl-1,3,4,6-tetrahydro-2,5-benzoxazocine, CAS registry number 13669-70-0), as well as its individual (-)-nefopam enantiomer shown below under formula (III) and mixture thereof.

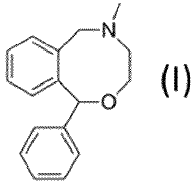

Nefopam free base may therefore be used as a mixture of its two enantiomers: (+)-nefopam and (-)-nefopam shown below under formula (II) and (III) respectively:

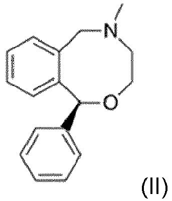

(S)-5-methyl-1-phenyl-1,3,4,6-tetrahydro-2,5-benzoxazocine ((+)-nefopam free base: CAS registry number 110011-82-0), and

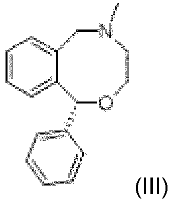

(R)-5-methyl-1-phenyl-1,3,4,6-tetrahydro-2,5-benzoxazocine ((-)-nefopam free base: CAS registry number 91463-82-0).

It must be understood that (-)-nefopam, or the racemic mixture (i.e. a ratio 1:1 of both enantiomers) taken separately can be used to fulfill the purposes of the invention.

The invention also concerns the use of the above defined composition and compounds as malodor and/or off-taste counteracting ingredients, preferably as malodor counteracting ingredients, e.g. to modify, suppress, reduce, decrease, mask or block the sensory perception of malodors and/or off-tastes, preferably malodors.

"Malodor" or "undesirable odor" refers to compounds generally offensive or unpleasant to most people.

"Off-taste", as defined herein, shall be given its ordinary meaning and shall include tastes that are undesired or unpleasant to most people.

"Odor blocking" refers to the ability of a compound to dull the human sense of smell.

"Odor masking" refers to the ability of a compound with a non-offensive or pleasant smell that is dosed such that it limits the ability to sense a malodorous compound.

Those of skill in the art will also recognize that nefopam may exist in many different protonation states, depending on, among other things, the pH of its environment. The invention concerns the use of the free base form of racemic nefopam and/or ((-)-nefopam.

The term "solvate" is used herein to describe a molecular complex comprising nefopam and one or more solvent molecules such as alcohols (for example, ethanol), ketones, esters, ethers, nitriles and the like. The term "hydrate" is employed when said solvent is water.

The term "polymorph" refers to the ability of nefopam or a salt, or solvate thereof to exist in more than one form or crystal structure.

The term "salts" as used herein means the salt forms which nefopam is able to form. Salts may be formed from acid addition of certain organic and inorganic acids to basic centers, typically amine. Examples of such appropriate acids include, for instance, inorganic acids such as hydrohalogen acids, e.g. hydrochloric or hydrobromic acid, sulfuric acid, nitric acid, phosphoric acid and the like; or organic acids such as, for example, acetic, propanoic, hydroxyacetic, 2-hydroxypropanoic, 2-oxopropanoic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclohexanesulfamic, salicylic (i.e. 2-hydroxybenzoic), p-aminosalicylic and the like. Salts of a hydrogen atom or an amino group include salts of organic carboxylic acids such as acetic, benzoic, lactic, fumaric, tartaric, maleic, malonic, malic, isethionic, lactobionic and succinic acids; organic sulfonic acids, such as methanesulfonic, ethanesulfonic, benzenesulfonic and p-toluenesulfonic acids; and inorganic acids, such as hydrochloric, sulfuric, phosphoric and sulfamic acids. It is to be understood that the compositions herein also comprise racemic nefopam and/or ((-)-nefopam may comprisecombinations with stoichiometric amounts of water as in hydrates. Preferable anions to form acid addition salts are acetate, benzenesulfonate , benzoate, bicarbonate, bitartrate, bromide, calcium edetate, camsyiate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methyl nitrate, methylsulfate, mucate, napsylate, nitrate, palmoate (embonate), pantothenate, phosphate/diphosphate, polygalacturonate, salicylate, stearate, subacetate, succinate, sulfate, tannate, tartrate, teoclate, triethiodide, and the like. In the present invention racemic nefopam free base, ((-)-nefopam free base, or a mixture thereof, devoid of any salt thereof is used.

The present invention also concerns methods to modify, suppress, reduce, decrease, mask and/or block the sensory perception of malodors or off-tastes, which comprise the step of contacting a situs such as air, a surface or the malodor source, with an effective amount of racemic nefopam free base, ((-)-nefopam free base, or a mixture thereof, or of a composition for use according to the invention.

According to any embodiment of the invention, the malodor or off-taste counteracted by racemic nefopam free base, ((-)-nefopam free base, or a mixture thereof, or a composition as described herein is of earthy, moldy and/or musty type. A non-exhaustive list of compounds presenting an earthy, moldy and/or musty note is shown in Table 1.

**Table 1**

| Common name | Chemical Name | References |
|---|---|---|
| Geosmin | 4,8a-dimethyl-1,2,3,4,5,6,7,8-octahydronaphthalen-4a-ol | ButteryR.G. and Garibaldi J.A. (1976) Geosmin and methylisoborneol in garden soil. J.Agric.Food Chem. 24,1246-7 |
| Patchouli Oil | | Sunderkötter A, Lorenzen S., Tacke R., Kraft P. (2010) Novel Silicon-Based Patchouli Odorants of the Trialkyl(1-hydroxy-1-methylethyl)silane Type: Design, Synthesis, and Olfactory Properties Chem. Eur. J. 16, 7404 -21 |
| 2-ethylfenchol | 6-ethyl-1,5,5-trimethylbicyclo[2.2.1]heptan-6-ol | Polak E, Trotier D et Baliguet (1978) Odor similarities in structurally related odorants Chem. Senses Flav. 3, 369-80 |
| 2-methylisoborneol | 1,2,7,7-Tetramethylbicyclo[2.2.1]heptan-2-ol | ButteryR.G. and Garibaldi J.A. (1976) Geosmin and methylisoborneol in garden soil. J.Agric.Food Chem. 24,1246-7 |
| Methyl Geosmin | 4,4,8a-trimethyl-1,2,3,4,5,6,7,8-octahydronaphthalen-4a-ol | Picco C., Gavazzo P., Firestein S., Mennini A. (1998) Response of isolated olfactory receptor sensory neurons to odorants. In Neuronal Circuits and Networks. Nato ASI series, series F Computer and Systems sciences Vol 167, pp85-93 |
| Huminol | 8-ethyl-1,5-dimethylbicyclo[3.2.1]octan-8-ol | Brunke E.-J. and Sruwe H. C-8-substituted 1,5-dimethyl-bicyclo [3.2.1]-octane-8-ols US patent 4,582,945 |
| Ambrinol S | (2R, 4aR)-(+)- 2,5,5-Trimethyl-1,2,3,4,4a,5,6,7-octahydro-naphthalen-2-ol | Naegeli P. and Win-Hahersack Y (1992) Enantiodifferentiation of odour perception of a-ambrinols Tetrahedron Assymetry 3, 221-2 |
| Ambrinol R | (2S, 4aS)-(-)- 2,5,5-Trimethyl-1,2,3,4,4a,5,6,7-octahydro-naphthalen-2-ol | Naegeli P. and Win-Hahersack Y (1992) Enantiodifferentiation of odour perception of a-ambrinols Tetrahedron Assymetry 3, 221-2 |
| Ambrinol racemic | 2,5,5-trimethyl-1,3,4,4a,6,7-hexahydronaphthalen-2-ol | Naegeli P. Process for the manufacture of alpha-ambrinol US patent 5,196,613 |

The present invention also encompasses the use as described herein of a consumer, industrial or textile product comprising racemic nefopam free base, ((-)-nefopam free base, or a mixture thereof, as described herein or a composition as described herein.

Racemic nefopam free base, ((-)-nefopam free base, or a mixture thereof, can be incorporated into all manners of composition and consumer, industrial or textile products that are designed to freshen, reduce, suppress or eliminate malodors and/or off-tastes and more particularly malodors containing an earthy or moldy and/or musty note.

Malodor or off-taste counteracting effective amount is understood to mean the amount of the racemic nefopam free base, ((-)-nefopam free base, or a mixture thereof, employed in a functional product that is organoleptically effective to abate a given malodor or off-taste while reducing the combined intensity of the odor or taste level, wherein the given malodor is present in air space or has deposited on a substrate or is present in a substance.

Typically, racemic nefopam free base, ((-)-nefopam free base, or a mixture thereof, may be employed in these compositions and products in varying amounts that will depend on the nature of the composition and consumer, industrial, or textile product and the particular end use application. Typically, however, one might expect to employ racemic nefopam free base, ((-)-nefopam free base, or a mixture thereof, in amounts ranging from 0.001% to 10% by weight, preferably 0.005% to 5.0% by weight, more preferably 0.01% to 2.0% by weight of the total composition, or consumer, industrial, or textile product.

In the preparation of a composition or product for use according to the present invention, the racemic nefopam free base, ((-)-nefopam free base, or a mixture thereof, may be combined with other ingredients typically employed. For example, in an air freshener, an additional malodor-counteracting active agent may be desirable. This might be, for example a perfume, a humidity absorber, or an anti-microbial active.

Typical anti-microbial actives include quaternary ammonium compounds (like cetyltrimethylammonium salts), chlorhexidine and salts thereof; diglycerol monocaprate, diglycerol monolaurate, glycerol monolaurate, polyhexamethylene biguanide salts (also known as polyaminopropyl biguanide salts-, an example being Cosmocil CQ available from Arch chemicals), 2,4,4'-trichloro, 2'-hydroxy-diphenyl ether (triclosan), and 3,7,11-trimethyldodeca-2,6,10-trienol (farnesol). Levels of incorporation may be from 0.01% to 1.0% by weight of the composition.

In addition, the composition or product may contain one or more excipients or adjuvants, which are typically included in such composition or products in order to aid in their manufacture, storage, application or performance.

In particular, said composition or product may contain a carrier material that may be hydrophobic or hydrophilic, solid or liquid depending on the nature of the composition or product and the use for which it is intended.

Hydrophobic carrier materials include silicones, that is, polyorganosiloxanes. Such materials may be cyclic or linear, examples include Dow Corning silicone fluids 344, 345, 244, 245, 246, 556, and the 200 series; Union Carbide Corporation Silicones 7207 and 7158; and General Electric silicone SF1202. Alternatively, or additionally, non-silicone hydrophobic materials may be used. Such materials include mineral oils, hydrogenated polyisobutene, polydecene, paraffins, isoparaffins of at least 10 carbon atoms, aliphatic or aromatic ester oils (eg. isopropyl myristate, lauryl myristate, isopropyl palmitate, diisopropyl sebecate, diisopropyl adipate, or C₈ to C₁₈ alkyl benzoates), and polyglycol ethers, for example polyglycol butanol ethers.

Hydrophilic carrier materials, for example water, may also be employed. Water can be distilled, deionized, or tap water. Water, containing a small amount of low molecular weight monohydric alcohols, e.g., ethanol, methanol, and isopropanol, or polyols, such as ethylene glycol and propylene glycol, can also be useful.

Organic solvents might also be employed. They include organic solvents that are aliphatic alcohols, for example monohydric or polyhydric alcohols having 2 to 8 carbon atoms, and polyglycol ethers, such as oligoglycol ethers having 2 to 5 repeat units. Examples include dipropylene glycol, glycerol, propylene glycol, butylene glycol, ethanol, propanol, isopropanol, and industrial methylated spirits.

Mixtures of carrier materials may also be used. The amount of carrier material employed is dependent on the nature of the product and can range from 1-99%, more typically 10-95%, by weight of a consumer product composition.

Structurants and emulsifiers may also be employed in the composition and consumer, industrial, or textile product of the invention. They may be present at levels from 0.01-50%, more typically 0.1-10% by weight of the composition or product.

Suitable structurants include cellulosic thickeners such as hydroxy propyl cellulose and hydroxy ethyl cellulose, and dibenzylidene sorbitol. Other suitable structurants include sodium stearate, stearyl alcohol, cetyl alcohol, hydrogenated castor oil, synthetic waxes, paraffin waxes, hydroxystearic acid, dibutyl lauroyl glutamide, alkyl silicone waxes, quaternium-18 bentonite, quatemium-18 hectorite, silica, and propylene carbonate. Suitable emulsifiers include steareth-2, steareth-20, steareth-21, ceteareth-20, glyceryl stearate, cetyl alcohol, cetearyl alcohol, PEG-20 stearate, dimethicone copolyol, and poloxamines.

Further emulsifiers/surfactants are perfume solubilizes and wash-off agents. Examples of the former include PEG-hydrogenated castor oil, available from BASF in the Cremaphor RH and CO ranges. Examples of the latter include poly(oxyethylene) ethers. Certain sensory modifiers are further desirable components in the compositions of the invention. Emollients, humectants, volatile oils, non-volatile oils, and particulate solids which impart lubricity are all suitable classes of sensory modifiers. Examples of such materials include cyclomethicone, dimethicone, dimethiconol, isopropyl myristate, isopropyl palmitate, talc, finely-divided silica (eg. Aerosil 200), polyethylene (eg. Acumist B18), polysaccharides, corn starch, C12-C15 alcohol benzoate, PPG-3 myristyl ether, octyl dodecanol, C7-C14 isoparaffins, di-isopropyl adipate, isosorbide laurate, PPG-14 butyl ether, glycerol, hydrogenated polyisobutene, polydecene, titanium dioxide, phenyl trimethicone, dioctyl adipate, and hexamethyl disiloxane.

The compositions, consumer, industrial, or textile products according to the invention may contain solubilizing aids for one or more other ingredients contained in said composition or product, including compounds according to formula (I) if needed. A suitable solubilizing aid is surfactant, preferably no-foaming or low-foaming surfactant. Suitable surfactants are nonionic surfactants, anionic surfactants, cationic surfactants, amphoteric surfactants, zwitterionic surfactants, and mixtures thereof, preferably nonionic surfactants and cationic surfactants, and mixtures thereof. Suitable solvents and diluents may be selected from compounds well known to the fragrance art for such purposes, typical non-limiting examples including propylene glycol, dipropylene glycol, isopropyl myristate, and the like.

Compositions and products according to the invention may contain preservatives, pH control agents (such as buffers), chelating agents, defoaming agents, antifoaming agents, antistatic agents, colorants, antioxidants, aesthetic agents such as opacifiers, perlizers, dyes and mixtures thereof.

If the composition or the product is in the form of an aerosol composition, a volatile propellant is typically employed. The level of incorporation of the volatile propellant is typically from 40-99% parts by weight.

Non-chlorinated volatile propellants are preferred, in particular liquefied hydrocarbons or halogenated hydrocarbon gases (particularly fluorinated hydrocarbons such as 1,1-difluoroethane and/or 1-trifluoro-2-fluoroethane) that have a boiling point of below 10 degrees Centigrade and especially those with a boiling point below zero degrees Centigrade. It is especially preferred to employ liquefied hydrocarbon gases, and especially C₃ to C₆ hydrocarbons, including propane, isopropane, butane, isobutane, pentane and isopentane and mixtures of two or more thereof. Preferred propellants are isobutane, isobutane/isopropane, isobutane/propane and mixtures of isopropane, isobutane and butane.

Other propellants that can be contemplated include alkyl ethers, such as dimethyl ether or compressed non-reactive gasses such air, nitrogen or carbon dioxide.

Fragrance can be also a desirable additional component in the composition and/or consumer product of the invention. Suitable materials include conventional perfumes, such as perfume oils and also include so-called deo-perfumes, as described in EP 545,556 patent and other publications. These latter materials may also qualify as additional organic anti-microbial agents. Levels of incorporation are preferably up to 20%, more typically up to 2% by weight. The fragrance ingredients may be selected for their odor characteristics, which are preferred in order to provide a fresh impression on the substrate to which the composition is directed.

Fragrance ingredients may be selected from the group consisting of aromatic and aliphatic esters having molecular weights from about 130 to about 250; aliphatic and aromatic alcohols having molecular weights from about 90 to about 240; aliphatic ketones having molecular weights from about 150 to about 260; aromatic ketones having molecular weights from about 150 to about 270; aromatic and aliphatic lactones having molecular weights from about 130 to about 290; aliphatic aldehydes having molecular weights from about 140 to about 200; aromatic aldehydes having molecular weights from about 90 to about 230; aliphatic and aromatic ethers having molecular weights from about 150 to about 270; and condensation products of aldehydes and amines having molecular weights from about 180 to about 320; and essentially free from nitromusks and halogenated fragrance materials.

Representative fragrance ingredients include: adoxal aliphatic aldehyde 2,6,10-trimethyl-9-undecen-1-al, allyl amyl glycolate, allyl amyl glycolate, allyl cyclohexane propionate, allyl-3-cyclohexyl propionate, amyl acetate, 3-methyl-1-butanol acetate, amyl salicylate, amyl salicylate, anisic aldehyde, 4-methoxy benzaldehyde, aurantiol, bacdanol, 2-ethyl-4-(2,2,3-trimethyl-3-cyclopenten-1-yl)-2-buten-1-ol, benzaldehyde, benzophenone, benzophenone, benzyl acetate, benzyl acetate, benzyl salicylate, benzyl salicylate, beta damascene, 1-(2,6,6-trimethyl-1-cyclohexen-1-yl)-2-buten-1-one, beta gamma hexanol, 3-hexen-1-ol, buccoxime, 1,5-dimethyl-oxime bicyclo[3,2,1] octan-8-one, cedrol, octahydro-3,6,8,8-tetramethyl-1H-3A,7-methanoazulen-6-ol, cetalox, dodecahydro-3A,6,6,9A- tetramethylnaphtho[2,1B]-furan, cis-3-hexenyl acetate, cis-3-hexenyl acetate, cis-3-hexenyl salicylate, beta, gamma-hexenyl salicylate, citronellol, 3,7-dimethyl-6-octenol, citronellyl nitrile, geranyl nitrile, clove stem oil, coumarin, cyclohexyl salicylate, cyclohexyl salicylate, cymal, 2-methyl-3-(para iso propylphenyl)propionaldehyde, decyl aldehyde, decyl aldehyde, delta damascene, 1-(2,6,6-trimethyl-3-cyclo-hexen- 1-yl)-2-buten-1-one, dihydromyrcenol, 3-methylene-7-methyl octan-7-ol, dimethyl benzyl carbinyl ester, dimethyl benzyl carbinyl acetate, ethyl vanillin, ethyl-2-methyl butyrate, ethyl-2-methyl butyrate, ethylene brassylate, ethylene tridecan-1,13-dioate, eucalyptol, 1,8-epoxy-para-menthane, eugenol alcohol 4-allyl-2-methoxy phenol, exaltolide, cyclopentadecanolide, flor acetate, dihydro-noryclopentadienyl acetate, florhydral, 3-(3-isopropylphenyl) butanal, frutene, dihydro-noryclopentadienyl propionate, galaxolide, 1,3,4,6,7,8-hexahydro-4,6,6,7,8,8-hexamethylcyclopentagamma-2-benzopyrane, gamma decalactone, 4-N-hepty-4-hydroxybutanoic acid, gamma dodecalactone, 4-N-octyl-4-hydroxy-butanoic acid, geraniol, 3,7-dimethyl-2,6-octadien-1-ol, geranyl acetate, 3,7-dimethyl-2,6-octadien-1-yl acetate, geranyl nitrile, 3,7-diemthyl-2,6-octadienenitrile, helional, alpha-methyl-3,4,(methylenedioxy) hydrocinnamaldehyde, heliotropin, heliotropin hexyl acetate, hexyl acetate, hexyl cinnamic aldehyde, alpha-n-hexyl cinnamic aldehyde, hexyl salicylate, hexyl salicylate, hydroxyambran, 2-cyclododecyl-propanol, hydroxycitronellal, hydroxycitronellal, ionone alpha, 4-(2,6,6-trimethyl-1-cyclohexenyl-1-yl)-3-buten-2-one, ionone beta aliphatic ketone 4-(2,6,6-trimethyl-1-cyclohexen-1-yl)-3-butene-2-one, ionone gamma methyl 4-(2,6,6-trimethyl-2-cyclohexyl-1-yl)-3-methyl-3-buten-2-one, iso E super 7-acetyl-1,2,3,4,5,6,7,8-octahydro-1,1,6,7,tetramethyl naphthalene, iso eugenol ether 2-methoxy-4-(1-propenyl) phenol, iso jasmone aliphatic ketone 2-methyl-3-(2-pentenyl)-2-cyclopenten-1-one, koavone, acetyl di-isoamylene, lauric aldehyde, lavandin natural, lavender natural, lemon CP, d-limonene, d-limonene/orange terpenes alkene 1-methyl-4-iso-propenyl-1-cyclohexene, linalool, 3-hydroxy-3,7-dimethyl-1,6-octadiene, linalyl acetate ester 3-hydroxy-3,7-dimethyl-1,6 octadiene acetate, 2,4-dihydroxy-3,6-dimethyl benzoic acid methyl ester, lyral, 4-(4-hydroxy-4-methylpentyl) 3-cylcohexene-1-carboxaldehyde, majantol, 2,2-dimethyl-3-(3-methylphenyl)-propanol, mayol, 4-(1-methylethyl) cyclohexane methanol, methyl anthranilate, methyl-2-aminobenzoate, methyl beta naphthyl ketone, methyl beta naphthyl ketone, methyl cedrylone, methyl cedrenyl ketone, methyl chavicol, 1-methyloxy-4,2-propen-1-yl benzene, methyl dihydrojasmonate, methyl dihydrojasmonate, methyl nonyl acetaldehyde, methyl nonyl acetaldehyde, musk indanone, 4-acetyl-6-tert butyl-1,1-dimethyl indane, nerol, 2-cis-3,7-dimethyl-2,6-octadien-1-ol, nonalactone, 4-hydroxynonanoic acid, norlimbanol, 1-(2,2,6-trimethyl-cyclohexyl)-3-hexanol, orange CP, P.T. bucinal, 2-methyl-3(para tert butylphenyl)propionaldehyde, para hydroxy phenyl butanone, para hydroxy phenyl butanone patchouli, phenyl acetaldehyde, 1-oxo-2-phenylethane phenyl acetaldehyde dimethyl, phenyl acetaldehyde dimethyl acetal phenyl ethyl acetate, phenyl ethyl acetate phenyl ethyl alcohol, phenyl ethyl alcohol, phenyl ethyl phenyl acetate, 2-phenylethyl phenyl acetate, phenyl hexanol/phenoxanol, 3-methyl-5-phenylpentanol, polysantol, 3,3-dimethyl-5-(2,2,3-trimethyl-3-cyclopenten-1-yl)-4-penten-2-ol, prenyl acetate, 2-methylbuten-2-ol-4-acetate, rosaphen, 2-methyl-5-phenyl pentanol, sandalwood, alpha-terpinene aliphatic alkane 1-methyl-4-iso-propylcyclohexadiene-1,3-terpineol (alpha terpineol and alcohol para-menth-1-en-8-ol, para-beta terpineol) menth-1-en-1-ol, terpinyl acetate ester para-menth-1-en-8-yl acetate, tetra hydro linalool, 3,7-dimethyl-3-octanol, tetrahydromyrcenol, 2,6-dimethyl-2-octanol, tonalid, 7-acetyl-1,1 ,3,4,4,6-hexamethyl, tetralin, undecalactone lactone 4-N-heptyl-4-hydroxybutanoic acid, undecavertol, 4-methyl-3-decen-5-ol, undecyl aldehyde, undecanal, undecylenic aldehyde, undecylenic aldehyde, vanillin aromatic aldehyde 4-hydroxy-3-methoxybenzaldehyde, verdox, 2-tert-butyl cyclohexyl acetate, vertenex, 4-tert-butyl cyclohexyl acetate and mixtures thereof.

Preferably, the compositions and products contain an effective amount of perfume to provide the freshening fragrance to surfaces when first applied and some lingering fragrance in-wear.

Racemic nefopam free base, ((-)-nefopam free base, or a mixture thereof, and/or the compositions for use in the present invention may be in the form of liquids or solids and they may be simply admixed into consumer, industrial, or textile product bases in these neat forms. Alternatively, if solid forms are used it may be desirable to solubilize them in a suitable solvent before mixing into the consumer, industrial, or textile product base.

Still further, it may be desired to spray dry a liquid racemic nefopam free base, ((-)-nefopam free base, or a mixture thereof, or the composition onto a solid support to form a powder, which can then be admixed with a consumer, industrial, or textile product base.

Still further, it may be desirable to present racemic nefopam free base, ((-)-nefopam free base, or a mixture thereof, or the compositions comprising racemic nefopam free base, ((-)-nefopam free base, or a mixture thereof, in a delivery vehicle. For example, it may be desirable to present racemic nefopam free base, ((-)-nefopam free base, or a mixture thereof, or the compositions comprising racemic nefopam free base, ((-)-nefopam free base, or a mixture thereof, on or in a solid support. Said vehicles may be in the form of a core-shell capsule, wherein the compound or composition is contained in a core and surrounded by a shell. Alternatively, it may be desired to absorb the racemic nefopam free base, ((-)-nefopam free base, or a mixture thereof, or the composition comprising racemic nefopam free base, ((-)-nefopam free base, or a mixture thereof, into a porous body such as a porous bead or the like. Further still, in may be desirable to dissolve or disperse racemic nefopam free base, ((-)-nefopam free base, or a mixture thereof, or the composition comprising racemic nefopam free base, ((-)-nefopam free base, or a mixture thereof, in a matrix material.

Such vehicles may be employed as a means of affecting the rate of release of racemic nefopam free base, ((-)-nefopam free base, or a mixture thereof, into the vapor phase, or it may act as a means by which racemic nefopam free base, ((-)-nefopam free base, or a mixture thereof, or a composition comprising racemic nefopam free base, ((-)-nefopam free base, or a mixture thereof, can be incorporated into a consumer, industrial, or textile product base. Still further, it may be a means of stabilizing racemic nefopam free base, ((-)-nefopam free base, or a mixture thereof, against an aggressive external environment.

Capsule shells may be formed from any of those materials conventionally known in the art and may include a naturally-derived material, such as gelatin or waxes; or a synthetic polymer, such as an aminoplast resin, an acrylic resin or a polyurea. Synthetic materials may include water-insoluble or water-soluble encapsulating materials such as polyethylenes, polyamides, polystyrenes, polyisoprenes, polycarbonates, polyesters, polyacrylates, vinyl polymers and polyurethanes and mixtures thereof.

Capsules may consist of a matrix of polysaccharide and polyhydroxy compounds, water-soluble or water-dispersible encapsulating materials comprising dextrins derived from ungelatinized starch acid-esters of substituted dicarboxylic acids; useful starches including, raw starch, pregelatinized starch, modified starch derived from tubers, legumes, cereal and grains, for example corn starch, wheat starch, rice starch, waxy corn starch, oat starch, cassava starch, waxy barley, waxy rice starch, sweet rice starch, amioca, potato starch, tapioca starch, oat starch, cassava starch, and mixtures thereof; modified starches including hydrolyzed starch, acid thinned starch, starch esters of long chain hydrocarbons, starch acetates, starch octenyl succinate, and mixtures thereof.

The term "hydrolyzed starch" refers to oligosaccharide-type materials that are typically obtained by acid and/or enzymatic hydrolysis of starches, preferably corn starch. Suitable hydrolyzed starches for inclusion in the present invention include maltodextrins and corn syrup solids. The hydrolyzed starches for inclusion with the mixture of starch esters have a Dextrose Equivalent (DE) values of from about 10 to about 36 DE. The DE value is a measure of the reducing equivalence of the hydrolyzed starch referenced to dextrose and expressed as a percent (on a dry basis). The higher the DE value, the more reducing sugars present. A method for determining DE values can be found in Standard Analytical Methods of the Member Companies of Corn Industries Research Foundation, 6th ed. Corn Refineries Association, Inc. Washington, DC 1980, D-52.

Starch esters having a degree of substitution in the range of from about 0.01% to about 10.0% may be used to encapsulate nefopam or a composition comprising nefopam.

Modified starches having emulsifying and emulsion stabilizing capacity such as starch octenyl succinates can be used. Preferably, the encapsulating material is water-soluble modified starch solid matrix, preferably a starch raw material that has been modified by treating said starch raw material with octenyl-succinic acid anhydride. More preferably the said modified starch is mixed with a polyhydroxy compound before treatment with octenyl-succinic acid anhydride.

A particular example of a modified starch is a waxy, maize starch, pregelatinized, dextrinized is mixed with sorbitol or any other alcohol type and then treated with octenyl succinic anhydride.

The use of such starches is described in European Patent 0 965 326.

Racemic nefopam free base, ((-)-nefopam free base, or a mixture thereof, or composition comprising racemic nefopam free base, ((-)-nefopam free base, or a mixture thereof, may be added to consumer, industrial or textile product bases as such, or they may be added to the bases as part of a mixture of ingredients. For example, racemic nefopam free base, ((-)-nefopam free base, or a mixture thereof, may be mixed with fragrance ingredients to form a fragrance composition, which can then be added to a consumer, industrial or textile product base. Racemic nefopam free base, ((-)-nefopam free base, or a mixture thereof, is odorless. It can be added to a perfume composition without influencing or affecting the particular hedonic effect that a perfumer is attempting to create.

In a particular embodiment of the invention, racemic nefopam free base, ((-)-nefopam free base, or a mixture thereof, can be used alone, in a composition as defined herein or in combination with other compounds, or added in a defined consumer, industrial, or textile product to counteract (i.e. modify, suppress, reduce, decrease, mask or block) a predominantly earthy, musty and/or moldy malodor present in closed environment such as a room, a cellar, a bathroom, a kitchen, a car interior where microorganisms develop due to the presence of water or to an elevated humidity level.

In another embodiment of the invention, racemic nefopam free base, ((-)-nefopam free base, or a mixture thereof, or a composition comprising racemic nefopam free base, ((-)-nefopam free base, or a mixture thereof, can be used alone or in combination with other compounds, or added in a defined consumer product as a food additive to modify, suppress, reduce, decrease, mask or block the earthy off note elicited by earthy tasting molecule such as geosmin or 2-methylisoborneol that can occur in food or beverages. For example, geosmin and 2-methylisoborneol accumulate in the flesh of fishes raised in pond or stagnant water, eliciting an unpleasant earthy or moldy off-taste. This off-taste can constitute a hurdle to the consumption of these fishes that otherwise represent a safe and valuable food source.

In another embodiment of the invention, racemic nefopam free base, ((-)-nefopam free base, or a mixture thereof, or a composition comprising racemic nefopam free base, ((-)-nefopam free base, or a mixture thereof, can be used alone in combination with other compounds, or added in a defined consumer or industrial product to modify, suppress, reduce, decrease or mask the malodor emanating from stagnant or waste waters such as water rejected by industries, water from sewage or in water treatment plants. According to this embodiment, racemic nefopam free base, ((-)-nefopam free base, or a mixture thereof, or a composition comprising racemic nefopam free base, ((-)-nefopam free base, or a mixture thereof, can be, alone or combination with other compounds or added to a consumer or industrial product, added to the waste water to be treated. Alternatively, it can be sprayed in the environment surrounding the said waste water.

In another aspect of the invention, racemic nefopam free base, ((-)-nefopam free base, or a mixture thereof, or a composition comprising racemic nefopam free base, ((-)-nefopam free base, or a mixture thereof, can be used alone or in combination with other compounds or added in a defined consumer, industrial or textile product to reduce the olfactory impact of earthy smelling compounds that are used for a particular purpose. More particularly, the earthy smelling compound can serve in integrated pest control. For example, earthy smelling compounds such, as but not restricted to, geosmin, an archetypal earthy smelling compound has been identified as a repellent for some insect pests (M.C. Stensmyr, in Cell, 151, 1345-1357, December 7, 2012; A.K. Wallingford in Pest Management Science, Volume 72, Issue 4, 701-706, April 2016). Racemic nefopam free base, ((-)-nefopam free base, or a mixture thereof, or a composition comprising racemic nefopam free base, ((-)-nefopam free base, or a mixture thereof, may therefore be useful to reduce the olfactory impact of earthy smelling compound geosmin for residents dwelt in the vicinity of cultures where earthy smelling compound geosmin is used as an insect repellent.

In a particular embodiment of the invention, racemic nefopam free base, ((-)-nefopam free base, or a mixture thereof, or a composition comprising racemic nefopam free base, ((-)-nefopam free base, or a mixture thereof, can be used to modify the olfactory character of a compound or a brand, used for perfume, flavor or fragrance composition that possess an earthy note. Since it has no intrinsic odor, racemic nefopam free base, ((-)-nefopam free base, or a mixture thereof, will not bring an additional note to the earthy compound or brand but will reveal or enhance the other olfactory notes owned by the compound or the brand to be modified. Racemic nefopam free base, ((-)-nefopam free base, or a mixture thereof, can therefore be used to repurpose smelling materials used in flavors and perfume creation.

The invention will now be illustrated by the following, non-limiting illustrations of particular embodiments of the invention.

### Examples

The following terms are used:
- h: hour
- RT: room temperature
- HCl: Hydrochloric acid
- NaHCO₃: Sodium bicarbonate
- LC-MS: Liquid Chromatography - Mass Spectrometry
- MS: Mass Spectrometry
- HPLC: High Performance Liquid Chromatography
- SOR: Specific Optical Rotation
- ESI: Electrospray lonisation
- NH₄OAc: Ammonium acetate
- min: minute
- ¹H NMR: Proton Nuclear Magnetic Resonance
- EtOAc: Ethyl Acetate
- DMSO: Dimethylsulfoxide
- DMSO-d6: Deuterated Dimethylsulfoxide
- DEA: Diethylamine
- TFA: trifluoroacetic acid
- ACN: Acetonitrile
- aq: aqueous
- CDCl₃: Deuterated Chloroform
- s: singleton
- d: doublet
- t: triplet
- q: quadruplet
- m: multiplet
- mL: Milliliters

### Example 1: Isolation of (S)-5-methyl-1-phenyl-1,3,4,6-tetrahydro-2,5-benzoxazocine ((+)-Nefopam):

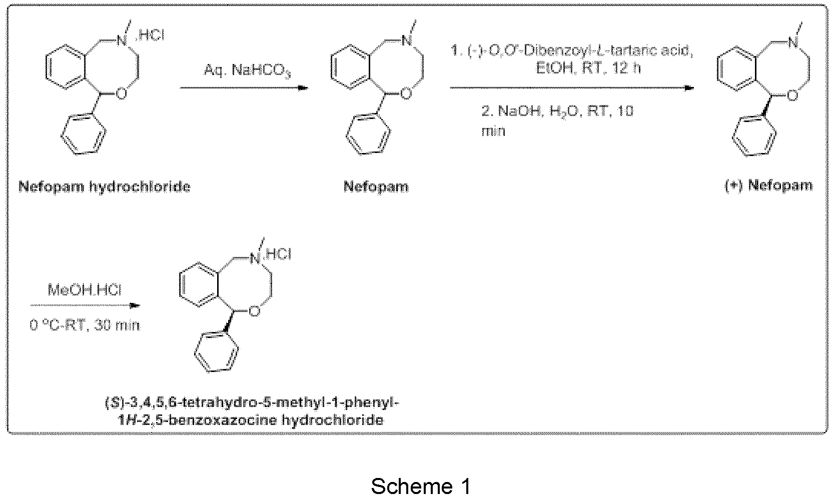

The isolation of (+)-nefopam was performed as shown in Scheme 1. A stirred solution of nefopam.HCl salt (250 g, 865.05 mmol; commercially available from ISOCHEM, Vert-le-petit, FRANCE) in water (250 mL) was basified with saturated aqueous NaHCO₃ solution (4 L) to a pH of about 7 and extracted with EtOAc (3 × 1 L). The combined organic extracts were dried over sodium sulfate, filtered and concentrated under reduced pressure to afford nefopam (200 g, 91%) as colorless oil.

To a stirred solution of nefopam (200 g, 790.51 mmol) in ethanol (200 mL) was added (-)-*O*,*O'-*dibenzoyl-L-tartaric acid (70.75 g, 197.63 mmol) in ethanol (200 mL) at RT and the mixture was stirred for 12 h. The obtained solid was collected by filtration, washed with ethanol (2 × 200 mL) and dried under vacuum to obtain 140 g of nefopam-L-tartaric acid salt as on off-white solid with 85.60% chiral HPLC purity.

The obtained Nefopam-L-tartaric acid salt was dissolved in ethanol (500 mL) and heated to 80 °C under stirring until dissolution of the solid (1 h). Then the mixture was gradually cooled to RT and allowed to stand for 5 h. The obtained crystals were collected by filtration, washed with ethyl acetate (2 × 250 mL) and dried under vacuum to afford nefopam-L-tartaric acid salt (160 g) as off white crystals with 91.16% chiral HPLC purity.

The above crystallization process was repeated twice to attain 100% chiral HPLC purity of Nefopam-L-tartaric acid salt (100 g, 21%) as off white crystals.

To a stirred solution of the obtained nefopam-L-tartaric acid salt crystals (100 g, 163.67 mmol) in water (250 mL) was added a solution of NaOH solution (16.37 g, 409.16 mmol) in water (250 mL) at RT and the mixture was stirred for 10 min. Then the mixture was diluted with water (200 mL) and extracted with ethyl acetate (3 × 200 mL). The combined organic extracts were dried over sodium sulfate, filtered and concentrated under reduced pressure to afford (+)-nefopam free base (40 g, 97%) as pale yellow oil.
¹H NMR (400 MHz, CDCl₃): δ 7.33-7.27 (m, 4H), 7.25-7.21 (m, 3H), 7.21-7.16 (m, 1H), 6.99 (d, J = 7.4 Hz, 1H), 5.80 (s, 1H), 4.81 (d, J = 12.7 Hz, 1H), 4.21-4.15 (m, 1H), 3.90-3.84 (m, 1H), 3.70 (d, J = 12.8 Hz, 1H), 2.86-2.80 (m, 1H), 2.66-2.60 (m, 1H), 2.46 (s, 3H);
LC-MS (Agilent 6310 Ion Trap): 99.07%; m/z 254.1 [M + H]⁺; (column; Kinetex EVO C-18 (50 × 3.0 mm, 2.6 µm); RT 2.93 min. 2.5 mM Aq. NH₄OAc : ACN; 0.8 mL/min);
HPLC (purity): 99.58%; (column: X SELECT CSH C18 (150 × 4.6 mm, 3.5 µm); RT 5.63 min; 0.05% TFA + 5% ACN : ACN + 5% 0.05% TFA; 1.0 mL/min, Diluent: H₂O : ACN); Chiral HPLC: Rt = 5.09 min (99.20%); (Chiralpak-IF3, 150 × 4.6 mm, 3.0 µm); mobile phase (A) 0.1% DEA in n-Hexane (B) CH₂Cl₂ : CH₃OH (50: 50) (A : B :: 95 : 05); flow Rate: 1.0 mL/min);
SOR: [α]20D +79.52 (c 0.25, CHCl₃); [α]20D +68.57 (c 0.25, EtOH).

Absolute stereochemistry confirmation of (+)-nefopam:
As (-)-nefopam HCl salt absolute stereochemistry was reported (Acta Chem. Scand. B 38 (1984) No. 4, 327-329), the above obtained (+)-nefopam was converted into HCl salt.

A mixture of (+)-Nefopam (50 mg, 0.19 mmol) free base in isopropyl alcohol (0.3 mL) under nitrogen atmosphere was heated to 50 °C and stirred for 30 min. To this solution was added concentrated HCl (0.03 mL) under the same temperature, and the mixture was stirred for 10 min. The mixture was cooled to 0 °C slowly. The precipitate obtained was filtered, washed with cold isopropyl alcohol (2 × 0.5 mL) and dried under vacuum to afford (+)-nefopam.HCl salt (7 mg, 12%) as an off-white solid.

¹H NMR (400 MHz, D₂O): δ 7.55-7.40 (m, 6H), 7.39-7.34 (m, 2H), 7.29 (br d, J = 7.3 Hz, 1H), 6.07 (s, 1H), 5.62 (br s, 1H), 4.53 - 4.44 (m, 1H), 4.39 (br d, J = 13.1 Hz, 1H), 4.21 (dt, J = 14.2, 3.5 Hz, 1H), 3.50 - 3.39 (m, 1H), 3.23 (dt, J = 14.6, 3.5 Hz, 1H), 3.05 (br s, 3H).

LC-MS: 96.01%; m/z 254.2 [M + H]+; (column; Ascentis Express C18, (50 × 3.0 mm, 2.7 µm); RT 1.79 min. 0.025% Aq. TFA + 5% ACN: ACN + 5% 0.025% Aq. TFA, 1.2 mL/min).

HPLC (purity): 98.70%; (column; X-Select CSH-C-18 (150 × 4.6 mm, 3.5 µm); RT 5.57 min. 0.05% TFA + 5% ACN: ACN + 5% 0.05% TFA; 1.0 mL/min, Diluent: ACN: H₂O).

Chiral HPLC: Rt = 13.53 min (99.27%); (Chiralcel-OJ-H, 250 × 4.6 mm, 5.0 µm); mobile phase (A) 0.1% DEA in n-Hexane (B) EtOH: MeOH (50: 50) (A : B :: 95 : 05); flow Rate: 1.0 mL/min);

Observed SOR for (+)-nefopam HCl salt: [α]20D +113.72 (c 0.1, DMSO); [α]20D +96.44 (c 0.7, MeOH).

Reported SOR for (-)-nefopam HCl salt: (Acta Chem. Scand. B 38 (1984) No. 4, 327-329) [α]^{22D} - 119 (c 1, DMSO); [α]20D -100.4 (c 0.7, MeOH).

From this it was confirmed that (+)-nefopam is an (*S*)-3,4,5,6-tetrahydro-5-methyl-1-phenyl-1H-2,5-benzoxazocine hydrochloride.

### Example 2: Isolation of (R)-5-methyl-1-phenyl-1,3,4,6-tetrahydro-2,5-benzoxazocine ((-)-Nefopam) :

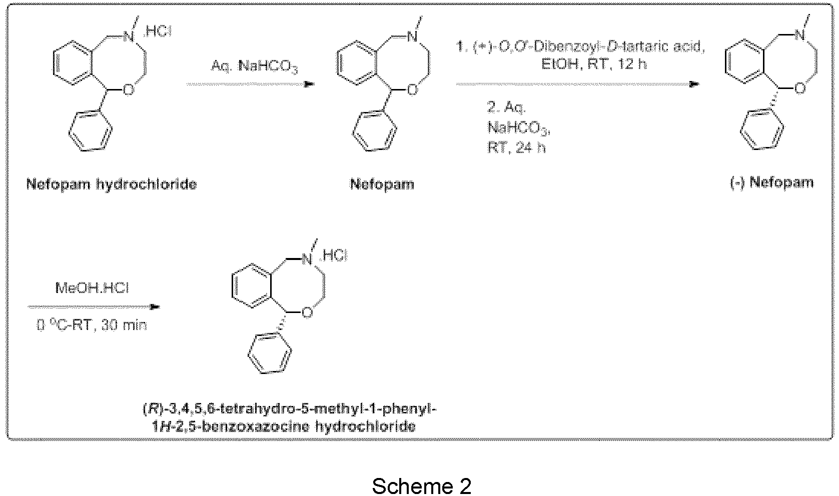

The isolation of (-)-nefopam was performed as shown in Scheme 2. A stirred solution of Nefopam.HCl salt (300 g, 1038.6 mmol) in water (1 L) was basified with saturated aqueous NaHCO₃ solution (6 L) to a pH of about 7 and extracted with ethyl acetate (3 × 500 mL). The combined organic extracts were dried over sodium sulfate, filtered and concentrated under reduced pressure to afford Nefopam (260 g, 99%) as colorless oil.

To a stirred solution of Nefopam (260 g, 1027.67 mmol) in ethanol (1 L) was added (+)-*O*,*O'-*dibenzoyl-D-tartaric acid (91.98 g, 256.92 mmol) in ethanol (500 mL) drop wise over a period of 20 min at RT, and the mixture was stirred for 12 h. The obtained solid was collected by filtration, washed with ethanol (2 × 200 mL) and dried under vacuum to obtain 180 g of nefopam-D-tartaric acid salt as on off white solid with 74.53% chiral HPLC purity.

The obtained nefopam-D-tartaric acid salt was dissolved in ethanol (800 mL), heated to 75°C under stirring until dissolution of the solid (4 h). Then the mixture was gradually cooled to RT and allowed to stand for 2 h. The obtained crystals were collected by filtration, washed with ethyl acetate (2 × 100 mL) and dried under vacuum to afford nefopam-D-tartaric acid salt (130 g) as off white crystals with 96.18% chiral HPLC purity.

The above crystallization process was repeated thrice to attain 98.15% chiral HPLC purity of Nefopam-D-tartaric acid salt (82 g) as off white crystals.

To a stirred solution of above obtained nefopam-D-tartaric acid salt crystals (82 g, 134.21 mmol) in water (1 L) was added aqueous saturated NaHCO₃ solution (2 L) at RT and the mixture was stirred for 24 h. Then the mixture was extracted with ethyl acetate (3 × 600 mL). The combined organic extracts were dried over sodium sulfate, filtered and concentrated under reduced pressure to afford (-)-nefopam free base (33 g, 97%) as pale yellow oil.
¹H NMR (400 MHz, DMSO-d6): δ 7.33-7.28 (m, 4H), 7.26-7.15 (m, 4H), 7.00-6.96 (m, 1H), 5.84 (s, 1H), 4.66 (d, J = 12.7 Hz, 1H), 4.01-3.95 (m, 1H), 3.78-3.72 (m, 1H), 3.68 (d, J = 12.7 Hz, 1H), 2.71-2.64 (m, 1H), 2.48-2.46 (m, 1H), 2.28 (s, 3H);
LC-MS (ESI): 99.66%; m/z 254.1 [M + H]⁺; (column; Kinetex EVO C-18 (50 × 3.0 mm, 2.6 µm); RT 2.10 min. 2.5 mM NH₄OOCH in water + 5% ACN : ACN + 5% 2.5 mM NH₄OOCH in water; 0.8 mL/min);
HPLC (purity): 99.76%; (column: X SELECT CSH C18 (150 × 4.6 mm, 3.5 µm); RT 9.63 min; 5 mM NH₄OAc: ACN; 1.0 mL/min, Diluent: ACN: H2O);
Chiral HPLC: Rt = 4.39 min (98.77%); (Chiralpak-IF3, 150 × 4.6 mm, 3.0 µm); mobile phase (A) 0.1% DEA in n-Hexane (B) CH₂Cl₂ : CH₃OH (50: 50) (A : B :: 95 : 05); flow Rate: 1.0 mL/min);
SOR: [α]20D -85.04 (c 0.25, CHCl3); [α]20D -93.79 (c 0.25, EtOH).

Absolute stereochemistry confirmation of (-)-nefopam:
As (-)-nefopam HCl salt absolute stereochemistry was reported (Acta Chem. Scand. B 38 (1984) No. 4, 327-329), the above obtained (-)-nefopam free base was converted into HCl salt.

A mixture of (-)-Nefopam (50 mg, 0.19 mmol) in isopropyl alcohol (0.3 mL) under nitrogen atmosphere was heated to 50 °C and stirred for 30 min. To this mixture was added concentrated HCl (0.03 mL) under the same temperature and the mixture was stirred for 10 min. The mixture was cooled to 0 °C. The precipitate obtained was filtered, washed with cold isopropyl alcohol (2 × 0.5 mL) and dried under vacuum to afford (-)-nefopam.HCl salt (25 mg, 43%) as an off-white solid.
¹H NMR (400 MHz, D₂O): 7.54 - 7.41 (m, 6H), 7.38 - 7.34 (m, 2H), 7.29 (br d, J = 7.4 Hz, 1H), 6.06 (s, 1H), 5.63 (br s, 1H), 4.56-4.33 (m, 2H), 4.21 (dt, J = 14.2, 3.5 Hz, 1H), 3.50- 3.39 (m, 1H), 3.23 (dt, J = 14.6, 3.5 Hz, 1H), 3.05 (br s, 3H);
LC-MS: 99.88%; m/z 254.2 [M + H]⁺; (column; Ascentis Express C18, (50 × 3.0 mm, 2.7 µm); RT 1.76 min. 0.025% Aq. TFA + 5% ACN: ACN + 5% 0.025% Aq. TFA, 1.2 mL/min).

HPLC (purity): 98.89%; (column: X SELECT CSH C18 (150 × 4.6 mm, 3.5 µm); RT 5.55 min; 0.05% TFA + 5% ACN: ACN + 5% 0.05% TFA; 1.0 mL/min, Diluent: ACN: H2O).

Chiral HPLC: Rt = 14.90 min (100.00%); (Chiralcel-OJ-H, 250 × 4.6 mm, 5.0 µm); mobile phase (A) 0.1% DEA in n-Hexane (B) EtOH: MeOH (50: 50) (A: B :: 95 : 05); flow Rate: 1.0 mL/min);
Observed SOR for (-)-Nefopam HCl salt: [α]20D -134.04 (c 0.1, DMSO); [α]20D -108.29 (c 0.7, MeOH).

Reported SOR for (-)-Nefopam HCl salt: (Acta Chem. Scand. B 38 (1984) No. 4, 327-329) [α]22D -119 (c 1, DMSO); [α]20D -100.4 (c 0.7, MeOH).

From this it was confirmed that (-)-nefopam is an (*R*)-3,4,5,6-tetrahydro-5-methyl-1-phenyl-1H-2,5-benzoxazocine hydrochloride.

### Example 3: Reduction of earthy olfactory note by a composition comprising racemic nefopam

A sensory test was used to demonstrate the effect of a racemic solution of nefopam (in its free base form) on the perception of the earthy olfactory note of different compounds. The composition comprising nefopam was prepared by diluting racemic nefopam in its free base form in ethanol at a concentration of 107 mg/ml

The test comprised in a first step, microinjecting the earthy compound, nefopam or earthy compound + nefopam in 20 L TedlarTM bags specially conceived for gas sampling, previously loaded with a known volume of pure air (from oil free compressor with additional charcoal filters). The earthy compounds were solubilized in ethanol at a suitable concentration (see table 2) and injected in the 20 L TeldarTM bag. The final concentration in the bag varied between 7 mg/m^{3,} to 383 mg/m³, (table 2), depending on the odor strength of the compound while the concentration of nefopam in the bags was fixed at 54 mg/m³.

**Table 2**

| | **Concentration of the liquid solution (mg/ml of ethanol)** | **Volume injected in test bag (µl)** | **Concentration of odorant in the bag (mg/m³ of air)** |
|---|---|---|---|
| Geosmin | 1.24 | 300 | 19 |
| Patchouli oil | 76.50 | 100 | 383 |
| 2-ethylfenchol | 12.50 | 300 | 188 |
| 2-methylisoborneol | 10.00 | 300 | 150 |
| Methyl geosmin | 13.50 | 10 | 7 |
| Huminol | 22.86 | 10 | 11 |
| Ambrinol S | 52.00 | 10 | 26 |
| Ambrinol R | 57.00 | 10 | 29 |
| Ambrinol racemic | 50.00 | 10 | 25 |
| Nefopam | 107.14 | 10 | 54 |

The odor presentation device comprised a cylinder filled with clean air. The bag was inserted in the cylinder and connected to a sampling installation in the cylinder by means of a valve. By controlled injection of clean air into the cylinder, the interior pressure was raised and the sampling air was pressed in a controlled manner out of the sample container through the opened valve. This sampling air was transferred to the nose of the corresponding sensory odor panel member by means of a funnel. By this means, each sensory odor panel member received an identical sample with a standardized volume flow and a constant provision time. The outlet flow rate was between 10-20 L/min (in order to avoid any mixing with ambient air before reaching the nose).

The expert panel of five assessors entered the room 15 minutes before starting the test. The basic standard test was a rating in intensity and the use of the closest chemical descriptors according to the following order of presentation:
1. earthy compound
2. Mix : earthy compound (at same concentrations as for 1) + Nefopam.

Each assessor could smell for few seconds. A pause of minimum 1 minute was considered between presentations.

The individual rating was done with a resolution of 0.5 on a scale ranging from 0 (no odor) to 5 (saturating odor).

The following results (Table 3) show that the typical earthy olfactory note associated with the tested compounds was strongly reduced when it was smelled in the presence of racemic nefopam. In contrast, the intensity of the other olfactory notes associated to the different compounds was not significantly decreased. This indicated that the odorless racemic solution of nefopam produced a strong attenuation/inhibition of the perception of the earthy note shared by different odorant molecules.

**Table 3**

| **Compound** | **Associated olfactory note** | **Odor intensity without Nefopam** | **Odor intensity with Nefopam** |
|---|---|---|---|
| Geosmin | note A : earthy | 3 | 1 |
| | note B : camphoreous | 3 | 2.5 |
| Patchouli Oil | note A : earthy | 2 | 0.5 |
| | note B : α-pinene | 3 | 2.5 |
| 2-ethylfenchol | note A : earthy | 3 | 0 |
| | note B : camphoreous | 3.5 | 3 |
| 2-methylisoborneol | note A : earthy | 3 | 0 |
| | note B : camphoreous | 0 | 2.5 |
| Methyl Geosmin | note A : earthy | 2.5 | 0.5 |
| | note B : α-pinene | 1.5 | 1.5 |
| Huminol | note A : earthy | 2.5 | 1 |
| | note B : camphoreous | 2.5 | 2 |
| Ambrinol S | note A : earthy | 2.5 | 0.5 |
| | note B : vetivert | 1.5 | 2 |
| Ambrinol R | note A : earthy | 1 | 0.5 |
| | note B : scatole | 3.5 | 3.5 |
| Ambrinol racemic | note A : earthy | 1.5 | 0 |
| | note B : α-pinene | 2.5 | 2.5 |

### Example 4: Effect associated to the enantiomers of nefopam.

Both enantiomers of nefopam, (+)-nefopam and (-)-nefopam in their free base from obtained as described in examples 1 and 2, respectively, were assessed separately as blockers of the perception of the earthy note of geosmin, using the same procedure as described in example 3. The results are shown in Table 4.

**Table 4**

| | | **Odor intensity** | | |
|---|---|---|---|---|
| **Compound** | **Associated olfactory note** | **without blocker** | **(-)-Nefopam** | **(+)-Nefopam** |
| Geosmin | note A : earthy | 3 | 0 | 3 |
| | note B : camphoreous | 3 | 3 | 3 |

| | | | | |
|---|---|---|---|---|
| (-)-Nefopam induced a strong reduction of the perception of the earthy note of geosmin for all the panelists. | | | | |

## Claims

1. Use of racemic nefopam free base, ((-)-nefopam free base, or a mixture thereof, or a polymorph or a solvate thereof, or use of a composition comprising racemic nefopam free base, ((-)-nefopam free base, or a mixture thereof, or a polymorph or a solvate thereof, as a malodor counteracting agent, wherein racemic nefopam free base is (*RS*)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

2. The use according to claim 1, wherein said malodor is earthy, musty and/or moldy malodor.

3. The use according to any one of claims 1 to 2, wherein said malodor is earthy malodor.

4. The use according to any one of claims 1 to 3, wherein (*R* )-5-methyl-1-phenyl-1,3,4,6-tetrahydro-2,5-benzoxazocine free base is used.

5. The use according to any one of claims 1 to 4, wherein the malodor causing agent comprises geosmin.

6. The use according to any one of claims 1 to 5, wherein the malodor causing agent comprises 2-methylisoborneol.

7. The use according to any one of claims 1 to 6, for blocking malodors.

8. A method of counteracting malodors comprising: contacting a situs comprising a malodor or a situs that will become malodorous with racemic nefopam free base, ((-)-nefopam free base, or a mixture thereof, or a polymorph or a solvate thereof, or contacting a situs comprising a malodor or a situs that will become malodorous with a composition comprising racemic nefopam free base, ((-)-nefopam free base, or a mixture thereof, or a polymorph or a solvate thereof, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

9. The method according to claim 8 wherein said malodor is earthy, musty and/or moldy malodors.

10. The method according to any one of claims 8 to 9, wherein the malodor causing agent comprises geosmin

11. The method according to any one of claims 8 to 10, wherein the malodor causing agent comprises 2-methylisoborneol.

12. Use of a consumer, industrial or textile product to freshen, reduce, suppress or eliminate malodors, wherein said product comprises racemic nefopam free base, ((-)-nefopam free base, or a mixture thereof, or a polymorph or a solvate thereof, or a composition comprising racemic nefopam free base, ((-)-nefopam free base, or a mixture thereof, or a polymorph or a solvate thereof, wherein racemic nefopam free base is (RS)-5-methyl-1-phenyl-1,3,4,6-tetrahydro-2,5-benzoxazocine free base, and ((-)-nefopam free base is (R)-5-methyl-1-phenyl-1,3,4,6-tetrahydro-2,5-benzoxazocine free base.

13. Use of a consumer, industrial or textile product according to claim 12 as an air-freshener.

## Patentansprüche

1. Verwendung von racemischem Nefopam, freie Base, ((-)-Nefopam, freie Base, oder eines Gemischs davon oder eines Polymorphs oder eines Solvats davon oder Verwendung einer Zusammensetzung umfassend racemisches Nefopam, freie Base, ((-)-Nefopam, freie Base, oder ein Gemisch davon oder ein Polymorph oder ein Solvat davon als ein schlechtem Geruch entgegenwirkendes Mittel, wobei racemisches Nefopam, freie Base, (RS)-5-Methyl-1-phenyl-1,3,4,6-tetrahydro-2,5-benzoxazocin, freie Base, ist und ((-)-Nefopam, freie Base, (R)-5-Methyl-1-phenyl-1,3,4,6-tetrahydro-2,5-benzoxazocin, freie Base, ist.

2. Verwendung gemäß Anspruch 1, wobei der schlechte Geruch erdiger, muffiger und/oder schimmeliger schlechter Geruch ist.

3. Verwendung gemäß einem der Ansprüche 1 bis 2, wobei der schlechte Geruch erdiger schlechter Geruch ist.

4. Verwendung gemäß einem der Ansprüche 1 bis 3, wobei (R)-5-Methyl-1-phenyl-1,3,4,6-tetrahydro-2,5-benzoxazocin, freie Base, verwendet wird.

5. Verwendung gemäß einem der Ansprüche 1 bis 4, wobei das schlechten Geruch verursachende Mittel Geosmin umfasst.

6. Verwendung gemäß einem der Ansprüche 1 bis 5, wobei das schlechten Geruch verursachende Mittel 2-Methylisoborneol umfasst.

7. Verwendung gemäß einem der Ansprüche 1 bis 6 zum Blockieren von schlechten Gerüchen.

8. Verfahren zum Entgegenwirken von schlechten Gerüchen, umfassend: Inkontaktbringen eines Situs, der einen schlechten Geruch umfasst, oder eines Situs, der schlechten Geruch umfassen wird, mit racemischem Nefopam, freie Base, ((-)-Nefopam, freie Base, oder einem Gemisch davon oder einem Polymorph oder einem Solvat davon oder Inkontaktbringen eines Situs, der einen schlechten Geruch umfasst, oder eines Situs, der schlechten Geruch umfassen wird, mit einer Zusammensetzung umfassend racemisches Nefopam, freie Base, ((-)-Nefopam, freie Base, oder ein Gemisch davon oder ein Polymorph oder ein Solvat davon, wobei racemisches Nefopam, freie Base, (RS)-5-Methyl-1-phenyl-1,3,4,6-tetrahydro-2,5-benzoxazocin, freie Base, ist und ((-)-Nefopam, freie Base, (R)-5-Methyl-1-phenyl-1,3,4,6-tetrahydro-2,5-benzoxazocin, freie Base, ist.

9. Verfahren gemäß Anspruch 8, wobei der schlechte Geruch erdige, muffige und/oder schimmelige schlechte Gerüche sind.

10. Verfahren gemäß einem der Ansprüche 8 bis 9, wobei das schlechten Geruch verursachende Mittel Geosmin umfasst.

11. Verfahren gemäß einem der Ansprüche 8 to 10, wobei das schlechten Geruch verursachende Mittel 2-Methylisoborneol umfasst.

12. Verwendung eines Verbraucher-, Industrie- oder Textilprodukts zum Frischmachen, Verringern, Unterdrücken oder Beseitigen von schlechten Gerüchen, wobei das Produkt racemisches Nefopam, freie Base, ((-)-Nefopam, freie Base, oder ein Gemisch davon oder ein Polymorph oder ein Solvat davon oder eine Zusammensetzung umfassend racemisches Nefopam, freie Base, ((-)-Nefopam, freie Base oder ein Gemisch davon oder ein Polymorph oder ein Solvat davon umfasst, wobei racemisches Nefopam, freie Base, (RS)-5-Methyl-1-phenyl-1,3,4,6-tetrahydro-2,5-benzoxazocin, freie Base, ist und ((-)-Nefopam, freie Base, (R)-5-Methyl-1-phenyl-1,3,4,6-tetrahydro-2,5-benzoxazocin, freie Base, ist.

13. Verwendung eines Verbraucher-, Industrie- oder Textilprodukts gemäß Anspruch 12 als Lufterfrischer.

## Revendications

1. Utilisation de la base libre du néfopam racémique, de la base libre du ((-)-néfopam ou d'un mélange correspondant, ou d'un polymorphe ou d'un solvate correspondant, ou utilisation d'une composition comprenant la base libre du néfopam racémique, la base libre du ((-)-néfopam ou un mélange correspondant, ou un polymorphe ou un solvate correspondant, en tant qu'agent contre les mauvaises odeurs, la base libre du néfopam racémique étant la base libre du (RS)-5-méthyl-1-phényl-1,3,4,6-tétrahydro-2,5-benzoxazocine, et la base libre du ((-)-néfopam étant la base libre du (R)-5-méthyl-1-phényl-1,3,4,6-tétrahydro-2,5-benzoxazocine.

2. Utilisation selon la revendication 1, lesdites mauvaises odeurs étant des mauvaises odeurs terreuses, de renfermé et/ou de moisi.

3. Utilisation selon l'une quelconque des revendications 1 à 2, lesdites mauvaises odeurs étant des mauvaises odeurs terreuses.

4. Utilisation selon l'une quelconque des revendications 1 à 3, la base libre du (R)-5-méthyl-1-phényl-1,3,4,6-tétrahydro-2,5-benzoxazocine étant utilisée.

5. Utilisation selon l'une quelconque des revendications 1 à 4, l'agent causant des mauvaises odeurs comprenant de la géosmine.

6. Utilisation selon l'une quelconque des revendications 1 à 5, l'agent causant des mauvaises odeurs comprenant du 2-méthylisobornéol.

7. Utilisation selon l'une quelconque des revendications 1 à 6, pour bloquer des mauvaises odeurs.

8. Procédé de lutte contre des mauvaises odeurs comprenant : la mise en contact d'un site comprenant des mauvaises odeurs ou d'un site qui va devenir malodorant avec la base libre du néfopam racémique, la base libre du ((-)-néfopam ou un mélange correspondant, ou un polymorphe ou un solvate correspondant, ou la mise en contact d'un site comprenant des mauvaises odeurs ou d'un site qui va devenir malodorant avec une composition comprenant la base libre du néfopam racémique, la base libre du ((-)-néfopam ou un mélange correspondant, ou un polymorphe ou un solvate correspondant, la base libre du néfopam racémique étant la base libre du (RS)-5-méthyl-1-phényl-1,3,4,6-tétrahydro-2,5-benzoxazocine, et la base libre du ((-)-néfopam étant la base libre du (R)-5-méthyl-1-phényl-1,3,4,6-tétrahydro-2,5-benzoxazocine.

9. Procédé selon la revendication 8, lesdites mauvaises odeurs étant des mauvaises odeurs terreuses, de renfermé et/ou de moisi.

10. Procédé selon l'une quelconque des revendications 8 à 9, l'agent causant des mauvaises odeurs comprenant de la géosmine.

11. Procédé selon l'une quelconque des revendications 8 à 10, l'agent causant des mauvaises odeurs comprenant du 2-méthylisobornéol.

12. Utilisation d'un produit de consommation, industriel ou textile pour rafraîchir, réduire, supprimer ou éliminer des mauvaises odeurs, ledit produit comprenant la base libre du néfopam racémique, la base libre du ((-)-néfopam ou un mélange correspondant, ou un polymorphe ou un solvate correspondant, ou une composition comprenant la base libre du néfopam racémique, la base libre du ((-)-néfopam ou un mélange correspondant, ou un polymorphe ou un solvate correspondant, la base libre du néfopam racémique étant la base libre du (RS)-5-méthyl-1-phényl-1,3,4,6-tétrahydro-2,5-benzoxazocine, et la base libre du ((-)-néfopam étant la base libre du (R)-5-méthyl-1-phényl-1,3,4,6-tétrahydro-2,5-benzoxazocine .

13. Utilisation d'un produit de consommation, industriel ou textile selon la revendication 12 en tant que désodorisant.
